# EUROPÄISCHE PATENTSCHRIFT

(11) **EP 1 395 207 B1**
(45) Veröffentlichungstag und Bekanntmachung des Hinweises auf die Patenterteilung: **27.09.2006**
(21) Anmeldenummer: 02740571.1
(22) Anmeldetag: 07.05.2002
(51) Int. Cl.: A61F 2/38

(54) **KNIE-ENDOPROTHESE**
KNEE ENDOPROTHESIS
ENDOPROTHESE DU GENOU

(30) Priorität: 18.05.2001 DE 20108394 U
(43) Veröffentlichungstag der Anmeldung: 10.03.2004
(73) Patentinhaber: Mathys AG Bettlach, 2544 Bettlach (CH)
(72) Erfinder: GLIEN, Wilfried, 07639 Bad Klosterlausnitz (DE); SALOMON, Dirk, 04626 Jonaswalde (DE); THON, Klaus, 07629 Hermsdorf (DE); RAHM, Jens, 08412 Werdau (DE); KATZER, Thomas, 07629 Hermsdorf (DE)
(74) Vertreter: Körfer, Thomas
(86) Internationale Anmeldenummer: PCT/EP2002/005004
(87) Internationale Veröffentlichungsnummer: WO 2002/094138

(56) Entgegenhaltungen:
- DE-U- 20 100 962
- FR-A- 2 663 536
- US-A- 5 928 286
- US-A- 6 123 728

## Beschreibung

Die Erfindung betrifft eine Knie-Endoprothese, mit einem Tibiaplateau, das eine plane Oberseite aufweist, mit einem Inlay als Meniskuskomponente und mit Femurkondylen, wobei die Oberseite des Inlays beim Beugen des Knies als Gleitlager für die Femurkondylen fungiert. In der Oberseite des Tibiaplateaus ist eine Bohrung zur Aufnahme und Fixierung eines Führungsbolzens zur Führung des Inlays vorgesehen, sowie eine Einfräsung, in die bei fixiertem Inlay eine Nase an der Unterseite des Inlays formschlüssig eingreift, wobei die Anordnung so getroffen ist, daß das Inlay drehbar oder drehfest mit dem Tibiaplateau verbunden sein kann.

Es gibt eine große Typenvielfalt von Knie-Endoprothesen, um die in jedem Einzelfall notwendigen Funktionen zu ersetzen, wobei die noch funktionstüchtigen natürlichen Bänder und Gelenkteile so weit wie möglich erhalten bleiben sollen. Grundsätzlich wird dabei zwischen Knie-Endoprothesen mit einem mobilen Inlay, das gegenüber dem Tibiaplateau beweglich ist, und Knie-Endoprothesen mit einem verriegelten Inlay, das am Tibiaplateau fixiert ist, unterschieden.

Aus EP-A-0 732 092 und EP-A-0 765 64 sind Knie-Endoprothesen bekannt, bei denen das Inlay nicht fest mit dem Tibiaplateau verbunden ist, sondern ihm gegenüber eine begrenzte Translations- und Rotationsbewegung ausführen kann.

Aus DE-A-43 08 563 ist es bekannt, das Inlay mittels einer Schwalbenschwanzführung auf dem Tibiaplateau festzulegen, so daß eine begrenzte Translations- und Rotationsbewegung möglich ist. Eine entsprechend ausgebildete Führung ist auch aus EP-A-0 634 156 bekannt.

Bei diesen Knie-Endoprothesen muß sich der Operateur vor dem Einsetzen des Tibiaplateaus entscheiden, ob er eine Knie-Endoprothese mit einem mobilen Inlay oder mit einem fixierten Inlay implantiert.

Aus EP-A-1 086 667 ist eine Knie-Endoprothese bekannt, bei der in der Lagerfläche des Tibiaplateaus eine Bohrung vorgesehen ist, in die ein Führungsbolzen für das Inlay einsetzbar ist. Die Bohrung und der Führungsbolzen sind so ausgebildet, daß der Führungsbolzen entweder drehfest oder drehbar gegenüber dem Tibiaplateau angebracht werden kann. Die Knie-Endoprothese kann dadurch aus einem Bausatz zusammengestellt werden, wobei intraoperativ noch die Wahlmöglichkeit zwischen mobilen Inlays unterschiedlicher Beweglichkeit besteht. Das Inlay kann drehbar und verschieblich, nur drehbar oder nur verschieblich sein, wobei jeweils dasselbe Tibiaplateau verwendet wird. Für verriegelte oder fixierte Inlays wird ein Tibiaplateau mit Vorsprüngen verwendet, die mit entsprechenden Hinterschneidungen am Inlay zusammenwirken.

Aus DE-U-201 00 962 ist eine Knie-Endoprothese der eingangs genannten Art bekannt, wobei bei beweglichem Inlay die Oberseite des Tibiaplateaus vollkommen plan ist und der Kopf des Führungsbolzens in einer Ausfräsung in der Unterseite des Inlays aufgenommen wird, wobei durch das Spiel zwischen Kopf und Ausfräsung das Ausmaß der Beweglichkeit des Inlays festgelegt ist. Nur bei verriegeltem Inlay hat die Oberseite des Tibiaplateaus die V-förmige Einfräsung, in die die nach unten zeigende Nase am anterioren Rand des Inlays eingreift. Mittels eines Gewindebolzens, der durch ein Langloch in den Inlay geht, kann das Inlay weitgehend festgelegt sein, so dass es keine Rotation und nur eine sehr begrenzte sagitale Verschiebung ausführen kann.

Die Aufgabe der Erfindung liegt in einer Knie-Endoprothese, bei der intraoperativ noch die Wahlmöglichkeit zwischen einem mobilen Inlay und einem fixierten Inlay besteht.

Erfindungsgemäß wird diese Aufgabe dadurch gelöst, daß entweder der Kopf des Führungsbolzens in einer Ausfräsung in der Unterseite des Inlays aufgenommen wird, wobei er das Ausmaß der Beweglichkeit des Inlays begrenzt, oder der Führungsbolzen das Inlay fixiert, wobei die Fixierung in Zusammenwirken mit dem Eingriff der Nase des Inlays in die Einfräsung des Tibiaplateaus erfolgt.
Dadurch, daß bei der erfindungsgemäßen Knie-Endoprothese das Tibiaplateau keine Erhebungen oder sonstige nach oben abstehende Komponenten hat, ist es mit mobilen Inlays mit einer glatten Unterseite ohne Vorsprünge kombinierbar. Außerdem ist dasselbe Tibiaplateau mit fixierten Inlays kombinierbar, die eine Nase oder ein sonstiges von der Unterseite vorspringendes Element aufweisen, das sich in die Einfräsung fügt. Die Einfräsung macht nur einen kleinen Anteil der Oberseite des Tibiaplateaus aus und seine Kanten sind abgerundet, so daß die Einfräsung bei einem mobilen Inlay nicht stört. Vorzugsweise hat die Einfräsung einen möglichst großen Abstand von der Bohrung des Tibiaplateaus. Insbesondere kann sie sich am Rand des Tibiaplateaus befinden.
Der Bolzen kann in die Bohrung des Tibiaplateaus eingeschraubt werden, so daß sein zylindrischer Kopf auf dem Tibiaplateau aufsitzt. Die Ausfräsung in der Unterseite des mobilen Inlays hat ein vorgegebenes Spiel gegenüber dem Durchmesser des Bolzenkopfes, wodurch das Ausmaß der Beweglichkeit des Inlays begrenzt wird. Bei allseitig geringem Spiel kann das Inlay gegenüber dem Tibiaplateau nur rotieren. Wird die Ausfräsung dagegen in sagittaler Richtung und/oder quer dazu erweitert, so kann sich das Inlay zusätzlich in diesen Richtungen verschieben.
Das Tibiaplateau kann auch in Verbindung mit einem fixierten Inlay verwendet werden. Ein solches Inlay wird an zwei Punkten fixiert, nämlich einmal durch die von der Unterseite des Inlays nach unten vorstehende Nase, die formschlüssig in der Einfräsung des Tibiaplateaus aufgenommen wird. Außerdem hat das Inlay eine Bohrung, die mit der Bohrung im Tibiaplateau fluchtet, jedoch einen größeren Durchmesser hat. In der Oberseite des Inlays ist eine die Bohrung umgebende Aussenkung ausgefräst. Mittels eines Gewindebolzens, der einen Schaft mit abgestuftem Durchmesser und einen Senkkopf hat, wird das Inlay am Tibiaplateau fixiert. Auf den Senkkopf des Bolzens folgt dabei zunächst ein glatter Abschnitt mit relativ großem Durchmesser, der in die Bohrung des Inlays paßt. Auf diesen Abschnitt folgt dann ein durchmesserreduzierter Gewindeabschnitt, der in dem Tibiaplateau festgeschraubt wird. Zwischen beiden Abschnitten besteht eine Stufe, die auf dem Tibiaplateau aufsitzt. Die Länge des glatten Abschnittes stimmt mit der Länge der Bohrung im Inlay überein. Die Länge des glatten Abschnittes und die Länge der Bohrung sind so aufeinander abgestimmt, daß der Kopf des Bolzens nur mit geringer Kraft das Inlay auf dem Tibiaplateau festspannt. Beim Festziehen des Bolzens wird nicht der Kopf des Bolzens gegen das Inlay gespannt, sondern die Stufe gegen das Tibiaplateau.

Dadurch, daß bei dem verriegeltem Inlay der abgestufte Gewindebolzen allenfalls mit geringer Kraft auf die Oberseite des Inlays drückt, wird eine Verformung des Inlays vermieden. Das Inlay besteht üblicherweise aus UHMW-PE. Würde der Gewindebolzen das Inlay festspannen, so würde sich das Inlay leicht schüsselartig verformen. Das Zentrum des Inlays um den Schraubenkopf herum würde komprimiert und die Ränder würden aufstehen. Außerdem würde bei einer zu hohen Druckspannung eine plastische Verformung des PE-Materials des Inlays stattfinden und eine Auslockerung des Gewindebolzens begünstigt.

Statt des abgestuften Gewindebolzens kann auch ein Aufsteckbolzen in die Bohrung eingeschraubt werden, auf den das Inlay mit enger Passung lediglich aufgesteckt wird. Der Aufsteckbolzen sitzt mit einem Bund auf dem Tibiaplateau auf und der Bund ist zweckmäßig mit Schlüsselflächen zum Ansetzen eines Schraubenschlüssels versehen. Der Aufsteckbolzen hat außerdem einen von der Oberseite des Tibiaplateaus nach oben abstehenden, relativ schlanken Schaft, auf den das Inlay aufgesteckt wird. Der Aufsteckbolzen kann mit umlaufenden Rippen oder einer sonstigen Profilierung versehen sein, durch die das Inlay auf dem Aufsteckbolzen gehalten wird und gegen ein Abheben von dem Tibiaplateau gesperrt wird.

Das Inlay kann in Abhängigkeit von den Erfordernissen des jeweiligen Falls einen in der Mitte der oberen Seite nach oben abstehenden Ansatz, die sogenannte Eminentia, aufweisen, der in einen zwischen den beiden Kondylen vorhandenen Dom ragt. Solche Knie-Endoprothesen werden als "posterior stabilized" und "semi-constraint" bezeichnet. Der Aufsteckbolzen kann bis in die Eminentia reichen und trägt dadurch noch zur Versteifung der Eminentia bei. Am posterioren Ende des Doms kann hierbei ein querverlaufender Stab vorhanden sein, der beim Beugen des Knies als Anschlag gegen die Eminentia läuft.

Die Fixierung eines Inlays erfolgt durch den abgestuften Gewindebolzen oder den Aufsteckbolzen in Verbindung mit einer an der Unterseite des Inlays vorstehenden Nase, die in der Aussparung oder Einfräsung in der Oberseite des Tibiaplateaus aufgenommen wird. Das Inlay wird dadurch in jedem Fall an zwei Punkten sicher fixiert.

Vorzugsweise ist die Bohrung im Tibiaplateau keine Gewindebohrung, sondern ist sie glatt und erweitert sich nach unten. Der Bolzen wird bei dieser Ausgestaltung in einen entsprechenden Klemmkonus eingeschraubt, der in das untere Ende der Bohrung des Tibiaplateaus eingesetzt wird. Auf diese Weise kann das Tibiaplateau auch mit unterschiedlichen Tibiaschäften verbunden werden, die ein entsprechend konisches proximales Ende aufweisen.

Das erfindungsgemäße Inlay besteht aus UHMW-PE und wird im allgemeinen in Verbindung mit einem Tibiaplateau aus einer bioverträglichen Titan- oder CoCrMo-Legierung oder einem bioverträglichen Keramikmaterial eingesetzt.

Das Inlay der erfindungsgemäßen Knie-Endoprothese ist vorzugsweise so ausgebildet, wie in DE-U-201 00962 beschrieben, wobei der posteriore Bereich der Oberseite des Inlays im Sagittalschnitt konvex geformt ist, wodurch ein aktiver Kippschutz erreicht wird.

Ein Vorteil der erfindungsgemäßen Knie-Endoprothese ist ihre mechanische Einfachheit und Stabilität, wobei eine Metall-Metall-Reibpaarung vermieden wird.

Ein Ausführungsbeispiel der Erfindung wird nachfolgend anhand der Zeichnung näher erläutert. Es zeigen:
- Fig. 1: im Schnitt die wesentlichen Bestandteile der Knie-Endoprothese bei gestrecktem Knie, wobei der Schnitt durch den tiefsten Punkt der Mulde des Inlays gelegt ist;
- Fig. 2: in Draufsicht das Tibiaplateau;
- Fig. 3: im Schnitt das Tibiaplateau und ein bewegliches Inlay;
- Fig. 4: im Schnitt das Tibiaplateau und ein fixiertes Inlay;
- Fig. 5: im Schnitt die Fixierung eines Inlays mittels eines in die Bohrung des Tibiaplateaus eingesetzten Konus und
- Fig. 6: im Schnitt die Fixierung des Inlays mittels eines in die Bohrung des Tibiaplateaus eingeschraubten Aufsteckbolzens.

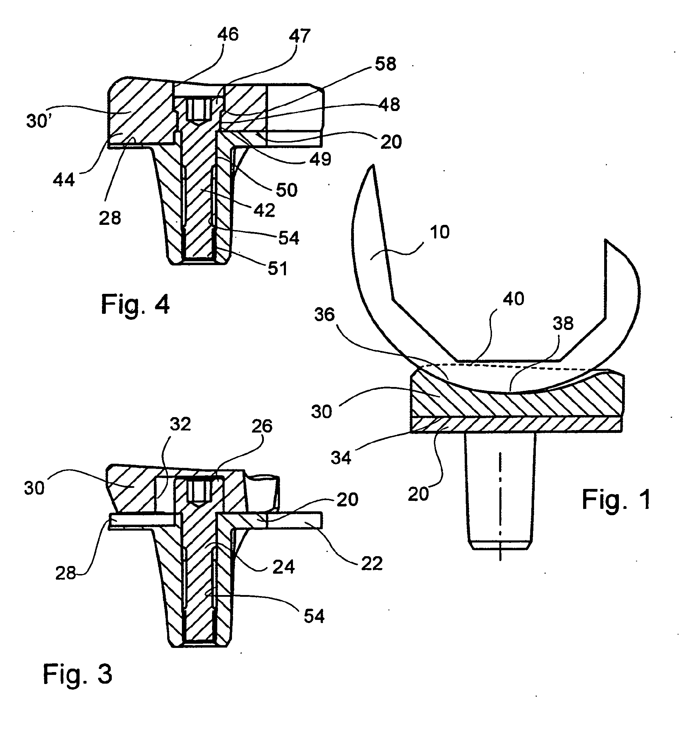

Fig. 1 zeigt eine Knie-Endoprothese mit Femurkondylen 10, einem Tibiaplateau 20 und dazwischen einem als Meniskus-Komponente fungierenden Inlay 30. In Fig. 1 ist die Endoprothese bei gestrecktem Knie dargestellt. Die Kondyle 10 wird am Femur verankert und das Tibiaplateau 20 wird an der Tibia verankert, wobei verschiedene Verankerungstechniken bekannt sind.

Das sich dazwischen befindende Inlay 30 kann in Abhängigkeit vom Aufbau der Prothese in einem vorgegebenen Ausmaß gegenüber dem Tibiaplateau 20 gleiten und rotieren (bewegliches Inlay) oder mit ihm fest verbunden sein (verriegeltes oder fixiertes Inlay).

Von oben betrachtet hat das Tibiaplateau 20 Nieren-Form mit einer halbkreisförmigen posterioren Aussparung 22 für das hintere Kreuzband und einer anterioren V-förmigen Einfräsung 28 (Fig. 2), die den Rand des Tibiaplateaus 20 berührt. Das in Fig. 3 dargestellte bewegliche Inlay 30 ist scheibenförmig und hat von oben betrachtet eine ähnliche Form wie das Tibiaplateau 20, doch etwas kleinere Abmessungen mit ebenfalls einer posterioren Aussparung für das hintere Kreuzband. In der Mitte des Tibiaplateaus 20 ist in eine Bohrung 54 ein Gewindebolzen 24 eingeschraubt, dessen zylindrischer Kopf 26 vorsteht. Der Gewindebolzen 24 dient zur Führung des Inlays 30. Das Inlay 30 hat in der Unterseite 34 eine längliche oder runde Ausfräsung 32, die den Kopf 26 mit Spiel aufnimmt. Die geometrischen Verhältnisse des Tibiaplateaus 20 zum Inlay 30 und des Kopfes 26 zur Ausfräsung 32 sind so gewählt, daß auch bei einer Beugung der Kondylen 10 das Inlay 30 frei, z.B. um ± 20°, gegenüber dem Tibiaplateau 20 rotieren kann und anteriorposterior etwa 9 mm und medial-lateral etwa 2 mm gleiten kann, ohne daß das Inlay 30 über das Tibiaplateau 20 übersteht. Die Oberseite 36 des Inlays 30 hat eine mediale und eine laterale konkave, flache Vertiefung oder Mulde 38 und eine Eminentia 40 zwischen den Mulden 38.

Die V-förmige Einfräsung 28 hat bei einem mobilen oder beweglichen Inlay 30 keine Funktion. Ihre Ränder sind mit einem Radius von 1 mm abgerundet und poliert, so daß sie die Bewegungen des Inlays 30 nicht behindern.

Bei dem beweglichen Inlay 30 finden Gleitbewegungen in zwei Ebenen statt, nämlich zum einen zwischen Tibiaplateau 20 und Inlay 30 und zum anderen zwischen Inlay 30 und Kondylen 10. Walkbewegungen zwischen Inlay 30 und Kondylen 10 werden dadurch minimiert, was die Lebensdauer des Implantats erhöht.

Fig. 4 zeigt ein Inlay 30', das verriegelt oder fixiert ist. Auch ein solches Inlay 30' ist scheibenförmig und hat von oben betrachtet eine ähnliche Form wie das Tibiaplateau 20 mit ebenfalls einer posterioren Aussparung für das hintere Kreuzband. Die Abmessungen eines verriegelten Inlays 30' entsprechen im allgemeinen denen des Tibiaplateaus 20. Die Unterseite 34 des Inlays 30 ist eben und hat anterior eine nach unten zeigende Nase 44, die in der V-förmigen Einfräsung 28 des Tibiaplateaus 20 liegt. Das Inlay 30' ist auf dem Tibiaplateau 20 mittels eines Gewindebolzens 42 weitgehend festgelegt, der durch eine Bohrung 46 in dem Inlay 30' geht und in die Bohrung 54 des Tibiaplateaus 20 eingedreht ist, so daß es keine Rotation und keine Verschiebung ausführen kann. Da das Inlay 30' fest mit dem Tibiaplateau 20 verbunden ist, findet beim Beugen des Knies eine Roll-Gleitbewegung, d.h. Walkbewegung, zwischen Inlay 30 und Kondylen 10 statt.

Der Gewindebolzen 42 hat einen Senkkopf 47, einen oberen glatten Schaft 48, eine Stufe 49, einen unteren glatten Schaft 50 und an der Spitze ein Gewinde 51. Der obere Schaft 48 hat einen deutlich größeren Durchmesser als der untere Schaft 50 und das Gewinde 51, so daß zwischen beiden die Stufe 49 vorhanden ist. Beim Festziehen des Gewindebolzens 42 liegt der Senkkopf 47 in einer entsprechend konischen Aussenkung 58 am oberen Ende der Bohrung 46 und die Länge des oberen Schafts 48 ist so bemessen, daß der Senkkopf 47 keinen wesentlichen Druck auf das Inlay 30' ausübt, wenn der Gewindebolzen 42 in das Tibiaplateau 20 eingeschraubt wird, wobei die Stufe 49 zwischen dem oberen Schaft 48 und dem unteren Schaft 50 gegen das Tibiaplateau 20 gespannt wird. Durch die konische Unterseite des Senkkopfes 47 wird erreicht, daß der vom Gewindebolzen 42 ausgeübte Druck sich nicht um die Bohrung 46 herum konzentriert, sondern sich auf einen größeren Bereich des Inlays 30' verteilt, so daß das Inlay 30' immer plan auf dem Tibiaplateau 20 aufliegt.

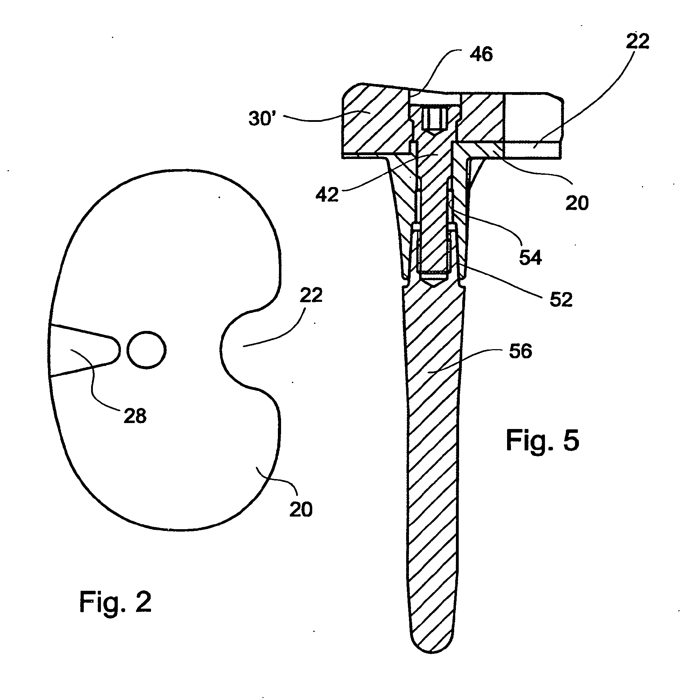

Das Gewinde, in das der Gewindebolzen 42 eingeschraubt wird, braucht nicht im Tibiaplateau 20 selbst vorgesehen zu sein. Der Gewindebolzen 42 kann in einen Konus 52 eingeschraubt werden, der in einer sich nach unten konisch erweiternden Bohrung 54 des Tibiaplateaus 20 eingesetzt ist. Wie in Fig. 5 gezeigt, kann das Tibiaplateau 20 dadurch mit einem Tibiaschaft 56 verbunden werden, wobei hier dann der Konus 52 das obere Ende des Tibiaschafts 56 ist.

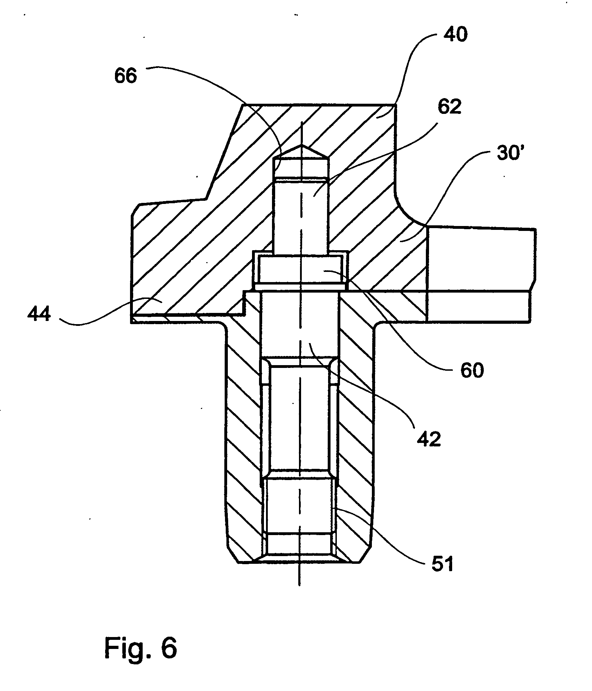

Bei dem Ausführungsbeispiel von Fig. 6 ist der Gewindebolzen 42 als Aufsteckbolzen ausgebildet. Der Gewindebolzen 42 ist mittels eines Gewindes 51 in das Tibiaplateau 20 eingeschraubt. Er kann auch entsprechend Fig. 5 mittels eines Konus 52 am Tibiaplateau 20 festgespannt werden. Der Gewindebolzen 42 hat einen Bund 60, der auf der Oberseite des Tibiaplateaus aufsitzt. An den Bund schließt sich nach oben ein schlanker Schaft 62 an. Auf den Schaft 62 ist das Inlay 30 mit einer Sackbohrung 66 aufgesteckt. Ein solches fixiertes Inlay 30 wird insbesondere bei posterior stabilisierten Knie-Endoprothesen eingesetzt, bei denen die Eminentia 64 des Inlays 30 nach oben gezogen ist, so daß sie in einen Dom zwischen den Kondylen 10 eingreift. Der Schaft 62 erstreckt sich in die Eminentia 64 und trägt zu deren Versteifung bei. Der Schaft 62 kann eine Oberflächengestaltung aufweisen, die einem Abheben eines einmal aufgesteckten Inlays 30 einen Widerstand entgegensetzt. Der Schaft 62 kann bspw. mit Rippen versehen sein, die im Axialschnitt ein nach unten gerichtetes Sägezahnprofil aufweisen und dadurch ein Abnehmen des aufgesteckten Inlays 30 verhindern. Das Inlay 30 kann auch aufschnappbar auf den Schaft 62 sein.

### Bezugszeichenliste

- 10: Kondylen
- 20: Tibiaplateau
- 22: Aussparung
- 24: Gewindebolzen
- 26: Kopf
- 28: Einfräsung
- 30: Inlay
- 32: Ausfräsung im Inlay
- 34: Unterseite
- 36: Oberseite
- 38: Mulde
- 40: Eminentia
- 42: Gewindebolzen
- 44: Nase
- 46: Bohrung
- 47: Senkkopf
- 48: oberer Schaft
- 49: Stufe
- 50: unterer Schaft
- 51: Gewinde
- 52: Konus
- 54: Bohrung
- 56: Tibiaschaft
- 58: Aussenkung
- 60: Bund
- 62: schlanker Schaft
- 66: Sackbohrung

## Patentansprüche

1. Knie-Endoprothese mit einem Tibiaplateau (20), das eine plane Oberseite aufweist, mit einem Inlay (30, 30') als Meniskuskomponente und mit Femurkondylen (10), wobei die Oberseite des Inlays (30, 30') beim Beugen des Knies als Gleitlager für die Femurkondylen (10) fungiert, wobei in der Oberseite des Tibiaplateaus (20) eine Bohrung (54) zur Aufnahme und Fixierung eines Führungsbolzens (24, 42) zur Führung des Inlays (30, 30') vorgesehen ist, wobei die plane Oberseite des Tibiaplateaus (20) eine Einfräsung (28) aufweist, in die bei fixiertem Inlay (30') eine Nase (44) an der Unterseite des Inlays (30') formschlüssig eingreift, **dadurch gekennzeichnet, dass** auch die Anordnung so getroffen ist, dass das Inlay (30, 30') drehbar mit dem Tibiaplateau (20) verbunden sein kann, und wobei entweder der Kopf (26) des Führungsbolzens (24) in einer Ausfräsung (32) in der Unterseite des Inlays (30) aufgenommen wird, wobei er das Ausmaß der Beweglichkeit des Inlays (30) begrenzt, oder der Führungsbolzen (42) das Inlay (30') fixiert, wobei die Fixierung in Zusammenwirken mit dem Eingriff der Nase (44) des Inlays (30') in die Einfräsung (28) des Tibiaplateaus (20) erfolgt.

2. Knie-Endoprothese nach Anspruch 1, **dadurch gekennzeichnet, daß** bei einem fixierten Inlay (30') der Bolzen (42) einen Kopf (47), einen glatten, oberen Schaft (48) und einen unteren Schaft (50) aufweist, daß der Durchmesser des glatten, oberen Schaftes (48) größer als der des unteren Schaftes (50) ist, so daß zwischen beiden eine Stufe (49) vorliegt, und daß die Länge des oberen Schaftes (48) so auf die Dicke des Inlays (30') abgestimmt ist, daß der Kopf (47) mit allenfalls geringer Kraft das Inlay (30') gegen das Tibiaplateau (20) spannt.

3. Knie-Endoprothese nach Anspruch 2, **dadurch gekennzeichnet, daß** der Kopf des Gewindebolzens (42) ein Senkkopf (47) ist und daß die Bohrung (46) des Inlays (30'), durch die der Führungsbolzen (42) eingesetzt wird, am oberen Ende eine entsprechende Aussenkung (58) hat.

4. Knie-Endoprothese nach einem der Ansprüche 1 bis 3, **dadurch gekennzeichnet, daß** die Bohrung (54) in dem Tibiaplateau (20) ein sich konisch erweiterndes unteres Ende aufweist, in das wahlweise ein Konus (52) oder ein entsprechend gegenkonisches oberes Ende eines Tibiaschaftes (56) einsetzbar ist, in den bzw. das der Führungbolzen (24, 42) einschraubbar ist.

5. Knie-Endoprothese nach Anspruch 1 oder 4, **dadurch gekennzeichnet, daß** der Führungsbolzen (42) ein Aufsteckbolzen ist, und daß das Inlay (30) eine Sackbohrung (66) aufweist, mittels der es auf den Gewindebolzen (42) aufsteckbar ist.

## Claims

1. Knee replacement having a tibial plateau (20) with a planar upper side, having an inlay (30, 30') as a meniscus component and having femoral condyles (10), wherein the upper side of the inlay (30, 30') acts as a sliding bearing for the femoral condyles (10) during bending of the knee, wherein provided in the upper side of the tibial plateau (20) is a bore (54) for receiving and fixing a guide bolt (24, 42) for guiding the inlay (30, 30'), wherein the planar upper side of the tibial plateau (20) has a milled recess (28), into which in the case of a fixed inlay (30') a lug (44) at the underside of the inlay (30') positively engages, **characterized in that** the arrangement is such that the inlay (30, 30') may also be connected rotatably to the tibial plateau (20), and wherein either the head (26) of the guide bolt (24) is received in a milled-out portion (32) in the underside of the inlay (30), wherein it limits the extent of mobility of the inlay (30), or the guide bolt (42) fixes the inlay (30'), wherein the fixing is effected in interaction with the engagement of the lug (44) of the inlay (30') into the milled recess (28) of the tibial plateau (20).

2. Knee replacement according to claim 1, **characterized in that**, in the case of a fixed inlay (30'), the bolt (42) comprises a head (47), a smooth upper shank (48) and a lower shank (50), that the diameter of the smooth upper shank (48) is larger than that of the lower shank (50), so that between both there is a step (49), and that the length of the upper shank (48) is tuned to the thickness of the inlay (30') in such a way that the head (47) with at most slight force clamps the inlay (30') against the tibial plateau (20).

3. Knee replacement according to claim 2, **characterized in that** the head of the threaded bolt (42) is a countersunk head (47) and that the bore (46) of the inlay (30'), through which the guide bolt (42) is inserted, has a corresponding depression (58) at the upper end.

4. Knee replacement according to one of claims 1 to 3, **characterized in that** the bore (54) in the tibial plateau (20) has a conically widening bottom end, into which either a cone (52) or a correspondingly inversely conical upper end of a tibial shank (56) is insertable, into which the guide bolt (24, 42) may be screwed.

5. Knee replacement according to claim 1 or 4, **characterized in that** the guide bolt (42) is a snap-on bolt, and that the inlay (30) has a blind hole (66), by means of which it may be snapped onto the threaded bolt (42).

## Revendications

1. Endoprothèse du genou comportant un plateau tibial (20) qui présente un côté supérieur plan, un inlay (30, 30') en tant que composant de ménisque et des condyles fémoraux (10), le côté supérieur de l'inlay (30, 30') faisant office de palier lisse pour les condyles fémoraux (10) lors de la flexion, dans laquelle on prévoit, sur le côté supérieur du plateau tibial (20), un perçage (54) destiné à recevoir et à immobiliser un boulon de guidage (24, 42) pour guider l'inlay (30, 30'), le côté supérieur plan du plateau tibial (20) présente un fraisage (28) dans lequel s'engage, par complémentarité de formes, lorsque l'inlay (30') est immobilisé, un nez (44) sur le côté inférieur de l'inlay (30'),
**caractérisée en ce que**
la disposition est conçue de manière à ce que l'inlay (30, 30') puisse être relié au plateau tibial (20) également de manière à pouvoir tourner et dans laquelle soit la tête (26) du boulon de guidage (24) est logée dans un fraisage extérieur (32) dans le côté inférieur de l'inlay (30), la tête limitant l'amplitude de la mobilité de l'inlay (30), soit le boulon de guidage (42) immobilise l'inlay (30'), l'immobilisation ayant lieu en coopération avec l'engagement du nez (44) de l'inlay (30') dans le fraisage intérieur (28) du plateau tibial (20).

2. Endoprothèse du genou selon la revendication 1, **caractérisée en ce que**, dans l'inlay (30') immobilisé, le boulon (42) présente une tête (47), une tige (48) supérieure lisse et une tige (50) inférieure, **en ce que** le diamètre de la tige (48) supérieure lisse est supérieur à celui de la tige (50) inférieure, de manière à ce qu'il y ait entre les deux un palier (49) et **en ce que** la longueur de la tige supérieure (48) soit adaptée à l'épaisseur de l'inlay (30') de façon à ce que la tête (47) serre l'inlay (30') contre le plateau tibial (20) avec une force éventuellement faible.

3. Endoprothèse du genou selon la revendication 2, **caractérisée en ce que** la tête du boulon fileté (42) est une tête fraisée (47) et **en ce que** le perçage (46) de l'inlay (30'), à travers lequel le boulon de guidage (42) est inséré, a un creux (58) correspondant sur l'extrémité supérieure.

4. Endoprothèse du genou selon l'une quelconque des revendications 1 à 3, **caractérisé en ce que** le perçage (54) présente, dans le plateau tibial (20) une extrémité inférieure s'élargissant de manière conique, dans laquelle au choix un cône (52) ou une extrémité supérieure proportionnellement contre-conique d'une tige tibiale (56) peut être inséré(e), dans lequel respectivement laquelle le boulon de guidage (24, 42) peut être vissé.

5. Endoprothèse du genou selon la revendication 1 ou 4, **caractérisée en ce que** le boulon de guidage (42) est un boulon emboîtable et **en ce que** l'inlay (30) présente un trou borgne (66) au moyen duquel il peut être emboîté sur le boulon fileté (42).
